# EUROPEAN PATENT SPECIFICATION

(11) **EP 1 575 625 B1**
(45) Date of publication and mention of the grant of the patent: **27.01.2010**
(21) Application number: 03814670.0
(22) Date of filing: 09.12.2003
(51) Int. Cl.: A61L 12/08, A45C 11/04, B65B 25/00

(54) **CONTACT LENS PACKAGES CONTAINING GLYCEROL MONOSTEARATE**
GLYCEROLMONOSTEARAT-HALTIGE VERPACKUNGEN FÜR KONTAKTLINSEN
EMBALLAGES DE LENTILLES DE CONTACT CONTENANT DU MONOSTEARATE DE GLYCEROL

(30) Priority: 23.12.2002 US 436109 P
(43) Date of publication of application: 21.09.2005
(60) Divisional application: 06076076.6
(73) Proprietor: Johnson & Johnson Vision Care, Inc., Jacksonville, FL 32256 (US)
(72) Inventor: PECK, James, Jacksonville, FL 32224 (US); DUBEY, Dharmesh, Jacksonville, FL 32256 (US); TOKARSKI, Michael, Ponte Vedra Beach, FL 32082 (US); ZHANG, Qiang, Annandale, NJ 08801 (US); LI, Yufu, Bridgewater, NJ 08807 (US); ARNOLD, Steven, Sparta, NJ 07871 (US)
(74) Representative: Tunstall, Christopher Stephen
(86) International application number: PCT/US2003/039017
(87) International publication number: WO 2004/060099

(56) References cited:
- EP-A- 0 367 513
- EP-A- 1 033 326
- WO-A2-02/41044
- GB-A- 2 078 760
- US-A- 4 100 309
- US-A- 4 981 657
- US-A1- 2002 069 896
- DATABASE WPI Section Ch, Week 197737 Derwent Publications Ltd., London, GB; Class A96, AN 1977-65891Y XP002287714 & JP 52 093398 A (TOA IYO DENSHI KK) 5 August 1977 (1977-08-05)

## Description

### FIELD OF THE INVENTION

This invention related to packages for storing contact lenses as well as methods of using and preparing these packages.

### BACKGROUND

Contact lenses have been used commercially to improve vision since the 1950s. At first contact lenses were made of hard materials, which were relatively easy to handle and package for use, but were uncomfortable for many patients. Later developments, gave rise to softer more comfortable lenses made of hydrophobic hydrogels, particularly silicone hydrogels. These lenses are very pliable, but due to this texture and their chemical composition, they present a number of problems with packaging.

Most contact lenses are packaged in individual blister packages having a bowl portion and a foil top, where the bowl portion is made from a hydrophobic material such as polypropylene. *See* U.S. Patent Nos. 4,691,820; 5,054,610; 5,337,888; 5,375,698; 5,409,104; 5,467,868; 5,515,964; 5,609,246; 5,695,049; 5,697,495; 5,704,468; 5,711,416; 5,722,536; 5,573,108; 5,823,327; 5,704,468; 5,983,608; 6,029,808; 6,044,966; and 6,401,915 for examples of such packaging, all of which are hereby incorporated by reference in their entirety. While polypropylene is resilient enough to withstand the sterilization steps of contact lens manufacture, this material has an affinity for contact lenses made of silicone hydrogels. When silicone hydrogels are packaged in polypropylene bowls, the lenses stick to the bowl and cannot be removed from the package without damaging the pliable lenses. Therefore is a need to prepare a contact lens package that has resilient properties, but does not stick to the final product. It is this need that is met by the following invention.

### BRIEF DESCRIPTION OF THE DRAWINGS

Figure 1 illustrates the data for Lens A in different packages
Figure 2 illustrates the data for Lens B in different packages
Figure 3 illustrates the data for Lens C in different packages

### DETAILED DESCRIPTION OF THE INVENTION

This invention relates to a package according to claim 1.

The contact lenses can provide optical correction or may be cosmetic. The preferred contact lenses for use in the invention are soft contact lenses made from silicone elastomers or hydrogels, which include but are not limited to silicone hydrogels, and fluorohydrogels. Soft contact lens formulations are disclosed in U.S. Pat. App. No. 60/318,536, entitled Biomedical Devices Containing Internal wetting Agents," filed on September 10, 2001 and its non-provisional counterpart of the same title, filed on September 6, 2002, US Patent No. 5,710,302, WO 9421698, EP 406161, JP 2000016905, U.S. Pat. No. 5,998,498, US Pat. App. No. 09/532,943, U.S. Patent No. 6,087,415, U.S. Pat. No. 5,760,100, U.S. Pat. No.5,776, 999, U.S. Pat. No. 5,789,461, U.S. Pat. No. 5,849,811, and U.S. Pat. No. 5,965,631. The particularly preferred contact lenses used in the invention are soft contact lenses made from etafilcon A, genfilcon A, lenefilcon A, polymacon, balafilcon A, lotrafilcon A. and silicone hydrogels as prepared in U.S. Pat. No. 5,998,498, U.S. Pat. App. No. 09/532,943, a continuation-in-part of US Pat App. No. 09/532,943, filed on August 30, 2000, U.S. Patent No. 6,087,415, U.S. Pat. No. 5,760,100, U.S. Pat. No.5,776, 999, U.S. Pat. No. 5,789,461, U.S. Pat. No. 5,849,811, and U.S. Pat. No. 5,965,631. The more particularly preferred contact lenses used in the invention are soft contact lenses, balafilcon A, lotrafilcon A, galyfilcon A, senofilcon A, or those made as described in U.S. Pat. App. No. 60/318,536, entitled Biomedical Devices Containing Internal wetting Agents," filed on September 10, 2001 and its non-provisional counterpart of the same title, filed on September 6, 2002. The most particularly preferred contact lenses are soft contact lenses made from either galyfilcon A or senofilcon A.

The term "molded base" refers to any polymer, rubber, or plastic that can be formed into a receptacle for medical devices, where the size and shape of the base are determined by the device and other considerations known those who are skilled in the art of making or designing molded bases. For example molded bases may be individual blister packages, secondary packages, or hydrating trays. The molded base may be prepared from any number of materials provided that those materials are compatible with the chemical and physical properties of the device. Examples of suitable materials include but are not limited to polypropylene, polyethylene, nylons, olefin co-polymers, acrylics, rubbers, urethanes, polycarbonates, or fluorocarbons. The preferred materials are metallocenes polymers and co-polymers made of polypropylene, polyethylene, having a melt flow range of about 15 g/10 minutes to about 44 g/10 minutes as determined by ASTM D-1238. With respect to the shape of the molded base, examples of suitably shaped bases are disclosed in the following patents which are hereby incorporated by reference in their entirety, U.S. Patent Nos. D 458,023; 4,691,820; 5,054,610; 5,337,888; 5,375,698; 5,409,104; 5,467,868; 5,515,964; 5,609,246; 5,695,049; 5,697,495; 5,704,468; 5,711,416; 5,722,536; 5,573,108; 5,823,327; 5,704,468; 5,983,608; 6,029,808; 6,044,966; and 6,401,915. As in the cited references, the molded based is sealed about the cavity that encloses the contact lens. Flexible cover sheets can be made from can be an adhesive laminate of an aluminum foil and a polypropylene film or any other extruded or co-extruded film that can be sealed to the top surface of the flange in order to form a hermetic seal for the contact lens and the solution. Further, the base can be formed by any of a number of known methods which include but are not limited to injection molding, transfer molding, skin packaging, blow molding, coinjection molding, film extrusion, or film coextrusion.

The term "solution" refers to any liquid medium in which a contact lens is stored. The preferred solutions are aqueous solutions contain physiological buffers. The particularly preferred solution is saline solution.

It is preferred that the molded base is transparent to the degree necessary to permit visual inspection, UV sterilization or both. The glycerol monostearate is preferably present at 2 weight percent.

Further, the invention includes a method according to claim 12.

Other have tried to address the problem of a medical device adhering to its packaging. For example U.S. Pat App. No. 09/942,347, entitled "Textured Contact Lens Package," filed on August 29, 2001 and U.S. Pat. App. No. 10/183,133, entitled "Contact Lens Packages,"filed on June 26, 2002 disclose solutions to this problem.

In order to illustrate the invention the following examples are included. These examples do not limit the invention.

### EXAMPLES

The following abbreviations are used below

| | |
|---|---|
| Ampacet 40604 | fatty acid amide |
| ATOFINA 3924CWZ | Finacene Nucleated polypropylene having a melt flow of 55g/10 minutes, ASTM D1238. This material contains an antistat and a lubricant |
| Atmer 163 | fatty alkyl diethanolamine Reg. No.107043-84-5 |
| Dow Siloxane MB50-321 | a silicone dispersion |
| Epolene E43-Wax, | maleic anhydride produced by Eastman Chemical |
| Erucamide | fatty acid amide Registry No. 112-84-5 |
| Exxon 1605 | Exxon Achieve, PP1605, a metallocene polypropylene having a melt flow of 32 g/10 minutes, ASTM D-1238 (L) |
| Exxon 1654 | Exxon Achieve, PP1654, a metallocene isotactic polypropylene having a melt flow of 16 g/10 minutes, ASTM D-1238 (L) |
| Fina EOD-001 | Finacene, a metallocene and isotactic polypropylene having a melt flow of 16g/10 minutes, ASTM D1238 |
| Flura | Registry No.7681-49-4 |
| Kemamide | fatty acid amide |
| Licowax | fatty acid amide |
| Mica | Registry No. 12001-26-2 |
| Nurcrel 535 & 932 | ethylene-methacrylic acid co-polymer resin Registry No. 25053-53-6 |
| Oleamide | fatty acid amide Registry No. 301-02-0 |
| polyHema | poly hydroxy ethylmethacylate having a molecular weight of greater than 1 MM Dalton |
| mPDMS | 800-1000 MW monomethacryloxypropyl terminated polydimethylsiloxane |
| Pluronic | polyoxypropylene-polyoxyethylene block co-polymer Registry No. 106392-12-5 |
| PVP | poly vinyl pyrrolidinone, wherein KD# refers to different known molecular weight distributions of poly vinyl pyrrolidinone |
| Simma 2 | 3-methacryloxy-2-hydroxypropyloxy)propylbis (trimethylsiloxy)methylsilane |
| Super-Floss anti block | slip/anti blocking agent, Registry No. 61790-53-2 |
| Tetronic | alkyoxylated amine 110617-70-4 |
| Zeospheres anti-block | slip/anti blocking agent |
| Lens Preparations | |
| Lens A | Acquafilcon A lenses coated with polyhema having a molecular weight of about 1,000,000. See U.S. Pat App. No. 09/957,299, entitled "Soft Contact Lenses," filed on September 20, 2001, Example 27. The coating method is disclosed in U.S. Pat. App. No. 09/921,192, entitled "Method for Correcting Articles by Mold Transfer," filed on August 2, 2001. |
| Lens B | Contact lenses prepared as described in U.S. Pat. App. No. 60/318,536, entitled Biomedical Devices Containing Internal wetting Agents," filed on September 10, 2001 and its non-provisional counterpart of the same title, filed on September 6, 2002, containing by weight percent 30% Simma 2, 19% mPDMS, 31% DMA, 6% PVP (MW 360,000), 0.8%EDGMA, 0.23% CGI81, 1.5% Norbloc, 11% PVP (MW 2,500), 0.02% Blue Hema, 0-2 ac PDMS, 29% t-amyl alcohol. |
| Lens C | Contact lenses prepared as described in U.S. Pat. App. No. 60/318,536, entitled Biomedical Devices Containing Internal wetting Agents," filed on September 10, 2001 and its non-provisional counterpart of the same title, filed on September 6, 2002, containing by weight percent 28% Simma 2, 31% mPDMS, 23.5% DMA, 7% PVP (MW 360,000), 1.5%TEDGMA, 0.98% CGI 1850, 2.0% Norbloc, 6 HEMA, 0.02% Blue Hema. |

### Example 1

### Preparation of Packages with Different Additives

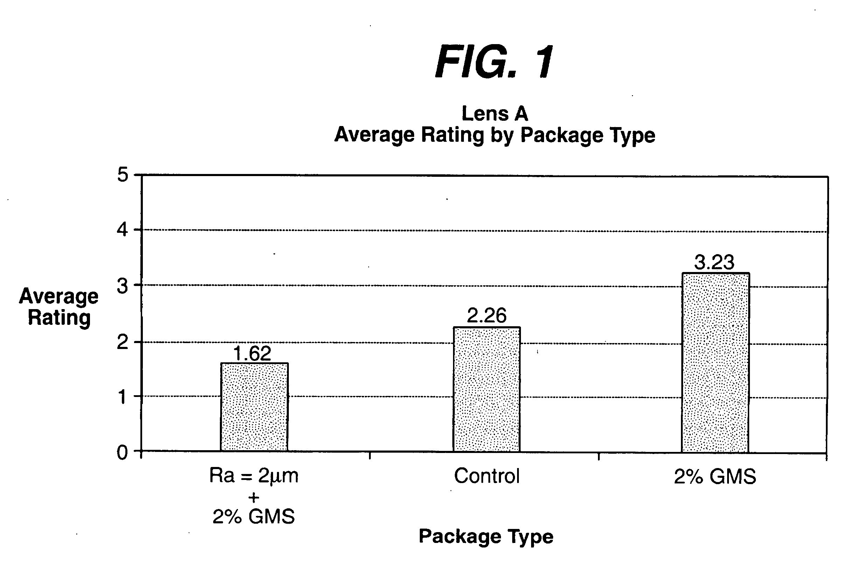

Additives (identity and amounts listed in Table 1) were mixed with polypropylene (listed below). The material was injection molded to form the base portion of a contact lens package. The configuration of the package is as illustrated in Figure1 of U.S. Pat No. 5,467,868 which is hereby incorporated by reference.

Contact lenses made from acquafilcon A coated with polyhema, a silicone hydrogel, were added to individual polypropylene blister packs having different additives containing 950µL of saline solution and then the blister pack was heat sealed with an flexible cover. Lenses were visually evaluated for adhesion to the package after sterilization. The flexible cover sheet was removed and the molded base is rotated or jiggled without spilling the saline solution while a contact lens is observed to determine if it is adhered to the inner surface of the molded base. Lenses that do not adhere are free floating and pass the test. If the lenses adhere to the molded base in any manner they fail the test. The addtitive, its weight percentage, the number of lenses that stuck to the package, and number of lenses that were free floating are displayed in Table 1. This example illustrates that glycerol monostearate is a superior additive.

**TABLE 1**

| Polypropylene | Additive | # tested | # stuck |
|---|---|---|---|
| Exxon 1605 | none | 12 | 12 |
| Exxon 1605 | calcium stearate | 36 | 36 |
| Exxon 1605 | 2% glycerol monostearate | 36 | 3 |
| Exxon 1654 | 2% glycerol monostearate | 84 | 2 |
| Exxon 1654 | none | 12 | 12 |
| Exxon Exxelor P1020 | none | 12 | 12 |
| Fina EOD-0011 | none | 12 | 12 |
| Fina EOD-0011 | 1% zinc stearate | 12 | 12 |
| Fina EOD-0011 | 3% zinc stearate | 12 | 12 |
| FINA 3924CW@ | antistat | 36 | 36 |

### Example 2

### Consumer Test

Packages containing 2% weight percent GMS and Exxon 1605 were prepared using the method of Example 1. Contact lenses of types A, B, and C were added to individual blister packages along with 950 µL of saline solution. The filed packages were heat sealed with flexible covers and sterilized. The packaged lenses were submitted to consumers. The consumers opened the packages and evaluated the lenses for ease of removal of the lens from the package using the following criteria and grading system
1-very easy removal-Lens comes out without any problems
2-easy removal-a couple of attempts to remove the lenses, but overall there were no real problems in removal
3-moderate removal- several tries before lens comes out, neither pleased or displeased
4-difficult removal-many tries to remove with finger or nail-removal is frustrating
5-very difficult removal-many tries to remove with a finger or nail, lens damage upon removal- very unacceptable

Figure 1 illustrates the testing results for a comparison of Lens A in a polypropylene package (control), Lens A in a package containing 2.0% GMS where the package has an average surface roughness (Ra) of about 2.0 µm, and Len A in a package containing 2.0% GMS. This figure shows that the roughened package containing GMS has the highest consumer rating.

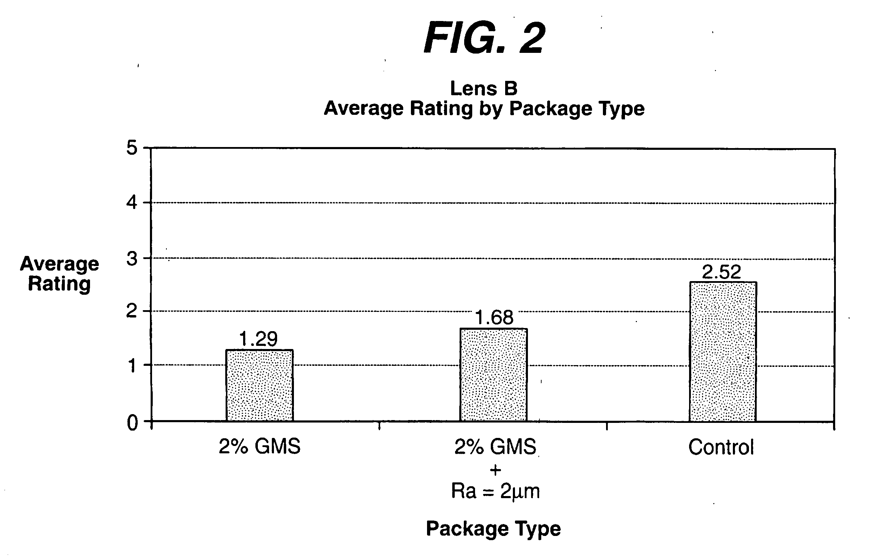

Figure 2 illustrates the testing results for a comparison of Lens B in a polypropylene package (control), Lens B in a package containing 2.0% GMS where the package has an average surface roughness (Ra) of about 2.0 µm, and Len B in a package containing 2.0% GMS. This figure shows that the package containing 2.0 %GMS has the highest consumer rating.

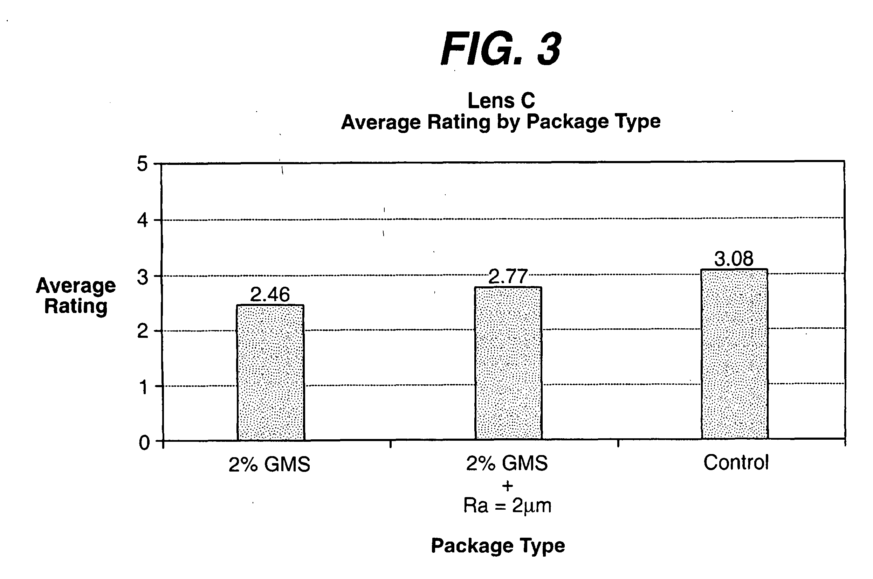

Figure 3 illustrates the testing results for a comparison of Lens C in a polypropylene package (control), Lens C in a package containing 2.0% GMS where the package has an average surface roughness (Ra) of about 2.0 µm, and Len C in a package containing 2.% GMS. This figure shows that the package containing 2.0 %GMS has the highest consumer rating.

### Example 3

### Preparation of Packages With Different Additives

The testing methods and preparations of Example 1 were repeated with different additives and lens types as per Table 2. If "(UP)" appears in an entry, that bowl of the blister is shaped as in U.S. Pat. No. D 458,023. When the term "Rough Bowl" appears, the inside surface of the bowl is roughened to an Ra of 0.5mm to 0.8mm.

**Table 2**

| Base Resin | Lens Type | Tested | Stuck | Additive |
|---|---|---|---|---|
| Exxon 1605 PP | Lens B | 15 | 13 | Calcium stearate (2%) |
| Exxon 1605 PP | Lens B | 120 | 0 | GMS (2%) |
| Exxon 1605 PP | Lens C | 30 | 0 | GMS (2%) |
| Exxon 1605 PP | Lens B | 15 | 12 | Dow Siloxane MB50-321 (10%) |
| Exxon 1605 PP | Lens B | 15 | 13 | Dow Siloxane MB50-321 (5%) |
| Exxon 1605 PP | Lens B | 57 | 50 | Ampacet 40604 99.5/.5 Erucamide |
| Ampacet 40604 PP | Lens B | 15 | 15 | Erucamide (5%) |
| Exxon 1605 PP | Lens B | 15 | 15 | Kemamide (Erucamide) (5%) |
| Exxon 1605 PP | Lens B | 15 | 12 | Superfloss anti-bock (2%) |
| Exxon 1605 PP | Lens B | 15 | 15 | Zeospheres anti-block (2%) |
| Exxon 1605 PP | Lens B | 15 | 14 | Superfloss anti-bock (2%) Oleamide (.2%) |
| Exxon 1605 PP | Lens B | 14 | 13 | Superfloss anti-bock (2%) Oleamide (.2%) |
| Exxon 1605 PP | Lens B | 15 | 15 | Talc (5%) |
| Exxon 1605 PP | Lens B | 15 | 13 | Calcium carbonate (5%) |
| Exxon 1605 PP | Lens B | 15 | 14 | Zinc stearate (5% hand blend) |
| Exxon 1605 PP | Lens B | 15 | 15 | Zinc stearate (5% machine blend) |
| Exxon 1605 PP | Lens B | 15 | 14 | ATP (Vitamin E) (5%) |
| Exxon 1605 PP | Lens B | 15 | 13 | Licowax (1%) |
| Exxon 1605 PP | Lens B | 15 | 14 | Polyethyleneglycol monolaurate (5%) |
| Exxon 1605 PP | Lens B | 15 | 15 | Mica (5%) |
| Exxon 1605 PP | Lens B | 175 | 8 | Succinic Acid (5%) |
| Exxon 1605 PP | Lens B | 15 | 13 | Succinic Anhydride (5%) |
| Exxon 1605 PP | Lens B | 118 | 22 | Epolene E-43 (20% machine blend) |
| Exxon 1605 PP | Lens B | 100 | 92 | Epolene E-43 (20% machine blend) |
| Exxon 1605 PP | Lens B | 127 | 52 | Epolene E-43 (10% hand blend) |
| Exxon 1605 PP | Lens B | 130 | 16 | Epolene E-43 (10% machine blend) |
| Exxon 1605 PP | Lens C | 15 | 6 | Epolene E-43 (10% machine blend) |
| Exxon 1605 PP | Lens B | 30 | 22 | Epolene E-43 (5% machine blend) |
| Exxon 1605 PP | Lens C | 15 | 3 | Epolene E-43 (5% machine blend) |
| Exxon 1605 PP | Lens B | 15 | 15 | Atmer 163 (1%) |
| Exxon 1605 PP | Lens B | 15 | 10 | MC (5%) |
| Exxon 1605 PP | Lens B | 30 | 2 | Boric Acid (5% hand blend) |
| Exxon 1605 PP | Lens B | 215 | 3 | Boric Acid (5% machine blend) |
| Exxon 1605 PP | Lens C | 15 | 0 | Boric Acid (5% machine blend) |
| Exxon 1605 PP | Lens B | 15 | 13 | Boric Acid (3% hand blend) |
| Exxon 1605 PP | Lens B | 15 | 15 | Boric Acid (2% hand blend) |
| Exxon 1605 PP | Lens B | 150 | 4 | Epolene E-43 (10% machine blend) |
| Exxon 1605 PP | Lens B | 50 | 9 | Epolene E-43 (10% machine blend) |
| Exxon 1605 PP | Lens B | 50 | 15 | Epolene E-43 (10% machine blend) |
| Exxon 1605 PP | Lens B | 50 | 35 | Epolene E-43 (10% machine blend) |
| Exxon 1605 PP | Lens B | 255 | 6 | PVP K90 (5.0%) |
| Exxon 1605 PP | Lens B | 98 | 31 | PVP K90 (2.5%) |
| Exxon 1605 PP | Lens B | 98 | 49 | PVP K90 (1.25%) |
| Exxon 1605 PP | Lens B | 20 | 6 | PVP K90 (1.0%) |
| Exxon 1605 PP | Lens B | 20 | 10 | PVP K90 (.75%) |
| Exxon 1605 PP | Lens B | 20 | 17 | PVP K90 (.5%) |
| Exxon 1605 PP | Lens C | 248 | 5 | PVP K90 (5.0%) |
| Exxon 1605 PP | Lens C | 39 | 0 | PVP K90 (10%) Blended down to 5% |
| Exxon 1605 PP | Lens C | 135 | 42 | PVP K90 (2.5%) |
| Exxon 1605 PP | Lens C | 135 | 54 | PVP K90 (1.25%) |
| Exxon 1605 PP | Lens C | 70 | 42 | PVP K90 (1.0%) |
| Exxon 1605 PP | Lens C | 70 | 50 | PVP K90 (.75%) |
| Exxon 1605 PP | Lens C | 70 | 60 | PVP K90 (.5%) |
| Exxon 1605 PP | Lens B | 15 | 14 | Nucrel 535 - 10.5% acid comonomer (2%) |
| Exxon 1605 PP | Lens B | 15 | 15 | Nucrel 925 - 15% add comonomer |
| (3%) | | | | |
| Exxon 1605 PP | Lens C | 15 | 14 | Nucrel 535 - 10.5% acid comonomer |
| (2%) | | | | |
| Exxon 1605 PP | Lens C | 15 | 14 | Nucrel 925 - 15% acid comonomer |
| (3%) | | | | |
| Exxon 1605 PP | Lens B | 15 | 15 | 2% XNAP with Pluronic |
| Exxon 1605 PP | Lens C | 15 | 14 | 2% XNAP with Pluronic |
| Exxon 1605 PP | Lens B | 15 | 15 | Pluronic 1% |
| Exxon 1605 PP | Lens C | 15 | 15 | Pluronic 1% |
| Exxon 1605 PP | Lens B | 15 | 11 | 1% Tetronic |
| Exxon 1605 PP | Lens C | 15 | 15 | 1 % Tetronic |
| Exxon 1605 PP | Lens B | 15 | 15 | 1% Flura |
| Exxon 1605 PP | Lens C | 15 | 15 | 1% Flura |
| Exxon 1605 PP | Lens B | 30 | 23 | 2% Pluronic |
| Exxon 1605 PP | Lens C | 30 | 16 | 2% Pluronic |
| Exxon 1605 PP | Lens C | 77 | 0 | PVP K90 (5%) + Epolene E43 (5%) |
| Exxon 1605 PP | Lens B | 50 | 0 | PVP K90 (5%) + Epolene E43 (5%) |
| Exxon 1605 PP | Lens C | 62 | 0 | PVP K90 (5%) + Epolene E43 (1.5%) |
| Exxon 1605 PP | Lens B | 50 | 0 | PVP K90 (5%) + Epolene E43 (1.5%) |
| Exxon 1605 PP | Lens C | 65 | 0 | PVP K90 (2.5%) + Epolene E43 (1.25%) |
| Exxon 1605 PP | Lens B | 50 | 0 | PVP K90 (2.5%) + Epolene E43 (1.25%) |
| Exxon 1605 PP | Lens C | 115 | 10 | PVP K90 (1%) + Epolene E43 (1%) |
| Exxon 1605 PP | Lens B | 100 | 11 | PVP K90 (1%) + Epolene E43 (1%) |
| Exxon 1605 PP | Lens C | 30 | 0 | PVP K29/31 (5%) |
| Exxon 1605 PP | Lens C | 30 | 0 | PVP K60 (5%) |
| Exxon 1605 PP | Lens B | 50 | 0 | PVP K90 (1%) + Rough Bowl (UP) |
| Exxon 1605 PP | Lens C | 50 | 0 | PVP K90 (1%) + Rough Bowl (UP) |
| Exxon 1605 PP | Lens B | 170 | 0 | Epolene E43 (1%) + Rough Bowl |
| Exxon 1605 PP | Lens C | 200 | 0 | Epolene E43 (1%) + Rough Bowl |

## Claims

1. A package for storing contact lenses in a solution comprising a molded base wherein the molded base comprises an additive, wherein the additive is glycerol monostearate, the package containing a contact lens in a solution, provided that the contact lens is not a contact lens consisting of acquafilcon A coated with polyHema.

2. The package of claim 1 wherein glycerol monostearate is present at a concentration of greater than 0.5 weight percent to 5 weight percent.

3. The package of claim 1 wherein glycerol monostearate is present at a concentration of 2 percent.

4. The package of claim 1 wherein the contact lens comprises balafilcon A, lotrafilcon A, galyfilcon, or senofilcon.

5. The package of claim 4 wherein the contact lens comprises Simma 2 and mPDMS.

6. The package of claim 4 wherein the contact lens comprises Simma 2.

7. The package of claim 1 wherein the molded base comprises polypropylene.

8. The package of claim 1 further comprising a cavity formed in said molded base wherein said cavity comprises an inner surface, wherein said inner surface has an average roughness of 0.5 µm to 20 µm.

9. The package of claim 8 wherein the inner surface has an average roughness of 1.8 µm to 4.5 µm.

10. The package of claim 8 wherein the inner surface has an average roughness of 1.9 µm to 2.1 µm.

11. The package of claim 8 wherein the inner surface has an average roughness of 0.5 µm to 0.8 µm.

12. A method of reducing the adherence of a contact lens to its packaging, comprising storing said contact lens in a solution in a package comprising a molded base wherein said molded base comprises an additive, wherein the additive is glycerol monostearate, provided that the contact lens is not a contact lens consisting of acquafilcon A coated with polyHema.

13. The method of claim 12 wherein glycerol monostearate is present at a concentration of greater than 0.25 weight percent to 5 weight percent.

14. The method of claim 12 wherein glycerol monostearate is present at a concentration of 2 percent.

15. The method of claim 12 wherein the contact lens comprises balafilcon A, or lotrafilcon A.

16. The method of claim 12 wherein the contact lens comprises Simma 2.

17. The method of claim 12 wherein the molded base comprises polypropylene.

18. The method of claim 12 further comprising a cavity formed in said molded base wherein said cavity comprises an inner surface, wherein said inner surface has an average roughness of 0.5 µm to 20 µm.

## Patentansprüche

1. Verpackung zur Aufbewahrung von Kontaktlinsen in einer Lösung, die eine ausgeformte Basis umfasst, wobei die ausgeformte Basis einen Zusatzstoff umfasst, wobei der Zusatzstoff Glycerolmonostearat ist, wobei die Verpackung eine Kontaktlinse in einer Lösung enthält, vorausgesetzt, dass die Kontaktlinse nicht eine Kontaktlinse ist, die aus mit PolyHema beschichtetem Acquafilcon A besteht.

2. Verpackung nach Anspruch 1, wobei Glycerolmonostearat in einer Konzentration von mehr als 0,5 Gewichtsprozent bis 5 Gewichtsprozent vorhanden ist.

3. Verpackung nach Anspruch 1, wobei Glycerolmonostearat in einer Konzentration von 2 Prozent vorhanden ist.

4. Verpackung nach Anspruch 1, wobei die Kontaktlinse Balafilcon A, Lotrafilcon A, Galyfilcon oder Senofilcon umfasst.

5. Verpackung nach Anspruch 4, wobei die Kontaktlinse Simma 2 und mPDMS umfasst.

6. Verpackung nach Anspruch 4, wobei die Kontaktlinse Simma 2 umfasst.

7. Verpackung nach Anspruch 1, wobei die ausgeformte Basis Polypropylen umfasst.

8. Verpackung nach Anspruch 1, die weiter einen Hohlraum umfasst, der in der ausgeformten Basis ausgebildet ist, wobei der Hohlraum eine Innenfläche umfasst, wobei die Innenfläche eine durchschnittliche Rauheit von 0,5 µm bis 20 µm besitzt.

9. Verpackung nach Anspruch 8, wobei die Innenfläche eine durchschnittliche Rauheit von 1,8 µm bis 4,5 µm besitzt.

10. Verpackung nach Anspruch 8, wobei die Innenfläche eine durchschnittliche Rauheit von 1,9 µm bis 2,1 µm besitzt.

11. Verpackung nach Anspruch 8, wobei die Innenfläche eine durchschnittliche Rauheit von 0,5 µm bis 0,8 µm besitzt.

12. Verfahren zur Verringerung des Anhaftens einer Kontaktlinse an ihrer Verpackung, das umfasst, dass die Kontaktlinse in einer Lösung in einer Verpackung aufbewahrt wird, die eine ausgeformte Basis umfasst, wobei die ausgeformte Basis einen Zusatzstoff umfasst, wobei der Zusatzstoff Glycerolmonostearat ist, vorausgesetzt, dass die Kontaktlinse nicht eine Kontaktlinse ist, die aus mit PolyHema beschichtetem Acquafilcon A besteht.

13. Verfahren nach Anspruch 12, wobei Glycerolmonostearat in einer Konzentration von mehr als 0,25 Gewichtsprozent bis 5 Gewichtsprozent vorhanden ist.

14. Verfahren nach Anspruch 12, wobei Glycerolmonostearat in einer Konzentration von 2 Prozent vorhanden ist.

15. Verfahren nach Anspruch 12, wobei die Kontaktlinse Balafilcon A oder Lotrafilcon A umfasst.

16. Verfahren nach Anspruch 12, wobei die Kontaktlinse Simma 2 umfasst.

17. Verfahren nach Anspruch 12, wobei die ausgeformte Basis Polypropylen umfasst.

18. Verfahren nach Anspruch 12, weiter umfassend einen Hohlraum, der in der ausgeformten Basis ausgebildet ist, wobei der Hohlraum eine Innenfläche umfasst, wobei die Innenfläche eine durchschnittliche Rauheit von 0,5 µm bis 20 µm besitzt.

## Revendications

1. Emballage destiné à stocker des lentilles de contact dans une solution, qui comprend une base moulée dans lequel la base moulée comprend un additif, dans lequel l'additif est du monostéarate de glycérol, l'emballage contenant une lentille de contact dans une solution, à condition que la lentille de contact ne soit pas une lentille de contact formée à partir d'acquafilcon A revêtu de polyHema.

2. Emballage selon la revendication 1, dans lequel le monostéarate de glycérol est présent en une concentration supérieure à 0,5 pour cent en poids et pouvant atteindre 5 pour cent en poids.

3. Emballage selon la revendication 1, dans lequel le monostéarate de glycérol est présent en une concentration égale à 2 pour cent.

4. Emballage selon la revendication 1, dans lequel la lentille de contact comprend du balafilcon A, du lotrafilcon A, du galyfilcon ou du senofilcon.

5. Emballage selon la revendication 4, dans lequel la lentille de contact comprend du Simma 2 et du mPDMS.

6. Emballage selon la revendication 4, dans lequel la lentille de contact comprend du Simma 2.

7. Emballage selon la revendication 1, dans lequel la base moulée comprend du polypropylène.

8. Emballage selon la revendication 1, comprenant en outre une cavité formée dans ladite base moulée dans laquelle ladite cavité comprend une surface intérieure, dans laquelle ladite surface intérieure présente une rugosité moyenne comprise entre 0,5 µm et 20 µm.

9. Emballage selon la revendication 8, dans lequel la surface intérieure présente une rugosité moyenne comprise entre 1,8 µm et 4,5 µm.

10. Emballage selon la revendication 8, dans lequel la surface intérieure présente une rugosité moyenne comprise entre 1,9 µm et 2,1 µm.

11. Emballage selon la revendication 8, dans lequel la surface intérieure présente une rugosité moyenne comprise entre 0,5 µm et 0,8 µm.

12. Procédé de réduction de l'adhérence d'une lentille de contact vis-à-vis de son emballage, qui comprend le stockage de ladite lentille de contact dans une solution dans un emballage qui comprend une base moulée dans lequel ladite base moulée comprend un additif, dans lequel l'additif est du monostéarate de glycérol, à condition que la lentille de contact ne soit pas une lentille de contact formée à partir d'acquafilcon A revêtu de polyHema.

13. Procédé selon la revendication 12, dans lequel le monostéarate de glycérol est présent en une concentration supérieure à 0,25 pour cent en poids et pouvant atteindre 5 pour cent en poids.

14. Procédé selon la revendication 12, dans lequel le monostéarate de glycérol est présent en une concentration égale à 2 pour cent.

15. Procédé selon la revendication 12, dans lequel la lentille de contact comprend du balafilcon A ou du lotrafilcon A.

16. Procédé selon la revendication 12, dans lequel la lentille de contact comprend du Simma 2.

17. Procédé selon la revendication 12, dans lequel la base moulée comprend du polypropylène.

18. Procédé selon la revendication 12, comprenant en outre une cavité formée dans ladite base moulée dans laquelle ladite cavité comprend une surface intérieure, dans laquelle ladite surface intérieure présente une rugosité moyenne comprise entre 0,5 µm et 20 µm.
